# EUROPEAN PATENT APPLICATION

(11) **EP 3 912 618 A1**
(43) Date of publication of application: **24.11.2021**
(21) Application number: 19910563.6
(22) Date of filing: 07.08.2019
(51) Int. Cl.: A61K 9/50, A61K 9/16, A61K 47/69

(54) **MICROCAPSULE COMPOSITION USING ALGINATE GEL, AND METHOD FOR PRODUCING SAME**

(30) Priority: 16.01.2019 KR 20190005726
(71) Applicant: Research Cooperation Foundation of Yeungnam University, Gyeongsan-si, Gyeongsangbuk-do 38541 (KR)
(72) Inventor: JEONG, Jee-Heon, Daegu 42010 (KR); PHAM, Thanh Tung, Gyeongsan-si Gyeongsangbuk-do 38541 (KR)
(74) Representative: Roos, Peter
(86) International application number: PCT/KR2019/009913
(87) International publication number: WO 2020/149481

(57) **Abstract**

The present invention relates to a microcapsule composition in which polydopamine-coated calcium carbonate microspheres are encapsulated by forming an alginate gel on the surface of a spheroid conjugated thereto, and a method of preparing the same, and it is confirmed that according to the method of preparing the microcapsule, the drug or physiologically active material was individually microencapsulated by gradually forming an alginate gel on the surface of the spheroid containing the drug or physiologically active material, a drug or a bioactive material is placed in the center of a capsule in a very simple way, and a capsule of a very small size can be manufactured in a short time compared to the conventional encapsulation method by adjusting the size of the capsule.

## Description

### [Technical Field]

The present invention relates to a microcapsule composition in which polydopamine-coated calcium carbonate microspheres are encapsulated by forming an alginate gel on the surface of a spheroid conjugated thereto, and a method of preparing the same.

### [Background Art]

Mesenchymal stem cells (MSCs), known as multipotent progenitor cells with indefinite cell division potential and multilineage differentiation ability, are known to promote angiogenesis and tissue regeneration, and to inhibit fibrosis and apoptosis and thus it is expected to be applicable to various regenerative medicine and transplantation treatment. However, since transplant surgery can induce an acute rejection response due to an immune response after transplantation, chronic immunosuppression through immunosuppressants or the like is required. To overcome this problem, microencapsulation technology has emerged as an alternative, but its clinical application is limited due to several problems.

First, MSCs are cultured as a two-dimensional monolayer which inadequately imitates their intrinsic microenvironment. Thus, long-term two-dimensional monolayer culture could negatively affect their replicative ability, colony-forming efficiency, and differentiation capabilities. To overcome this issue, three-dimensional spheroids of MSCs have been proposed to allow for better complex spatial cell-cell interactions and cell-extracellular matrix interaction, resulting in superior stemness properties and a higher therapeutic potential.

Second, the conventional encapsulation technology can produce a microgel containing a large number of cells or blank capsules which cannot encapsulate the cells inside the capsule, making it difficult to manufacture and handle encapsulated cells with a certain quality, and thus there is a problem of resulting in suboptimal therapeutic effects

Third, the conventional encapsulation technology generally has a large size (500 µm to 3 mm) of the manufactured capsule, so that the supply of oxygen and nutrients after encapsulation may not be smooth.

Fourth, the conventional encapsulation technologies lack of controls for the thicknesses of encapsulating layers desired by the producer, and during encapsulation, there are many cases in which capsules are produced that are not centered in the interior of the capsule and are skewed towards one side. This may cause differences in immunosuppressive effects, and may result in inconsistent therapeutic effects.

As a method to solve these problems, microencapsulation technology for protecting cells and spheroids of cells from the host immune system is emerging as an alternative, but recently encapsulation technology generally has problems of a low cell content in the capsule, increased transplantation mass, and the unstable control in the thickness of the encapsulated capsule.

Accordingly, there is a need to develop technology for individual encapsulation of drugs or bioactive materials, including cells, more effectively.

### [Disclosure]

### [Technical Problem]

The present invention relates to a method for individual encapsulation of an object, and provides a microcapsule composition in which the surface of the spheroid containing the object is coated with polydopamine, microspheres made of a material containing divalent cations are conjugated, and an alginate gel is formed and encapsulated on the surface of the spheroid to which the microspheres are conjugated.

### [Technical Solution]

The present invention provides a composition for microcapsules, which comprises: an object; microspheres conjugated to the object and composed of a material containing divalent cation; and alginate gel surrounding outside of the object and the microsphere, wherein the alginate gel is formed through a chelate bond between the divalent cation released from the material containing the divalent cation and alginate.

Also, the present invention provides a method of preparing microcapsules comprising: preparing microspheres composed of a material containing divalent cation (first step); coating surface of the microspheres with polydopamine by mixing a microspheres solution in which the microspheres are suspended and a dopamine solution (second step); conjugating polydopamine-coated microspheres (PD-MS) to surface of an object (third step); and coating surface of PD-MS-conjugated object with an alginate gel (fourth step).

In addition, the present invention provides an individual encapsulation method of an object comprising: preparing microspheres composed of a material containing divalent cation (first step); coating surface of the microspheres with polydopamine by mixing a microspheres solution in which the microspheres are suspended and a dopamine solution (second step); conjugating polydopamine-coated microspheres (PD-MS) to surface of an object (third step); and coating surface of PD-MS-conjugated object with an alginate gel (fourth step).

### [Advantageous Effects]

According to the present invention, it is confirmed that calcium carbonate microspheres conjugated to the surface of a spheroid containing a drug or a bioactive material release calcium ions in the alginate solution and chelate with alginate in the solution, thereby Individually microencapsulating drugs or bioactive materials by gradually forming alginate gel on the spheroid surface and thus the present invention can provide a method of preparing microcapsules and individual encapsulation method in which a drug or a bioactive material is placed in the center of a capsule in a very simple way, and a capsule of a very small size can be manufactured in a short time compared to the conventional encapsulation method by adjusting the size of the capsule.

### [Description of Drawings]

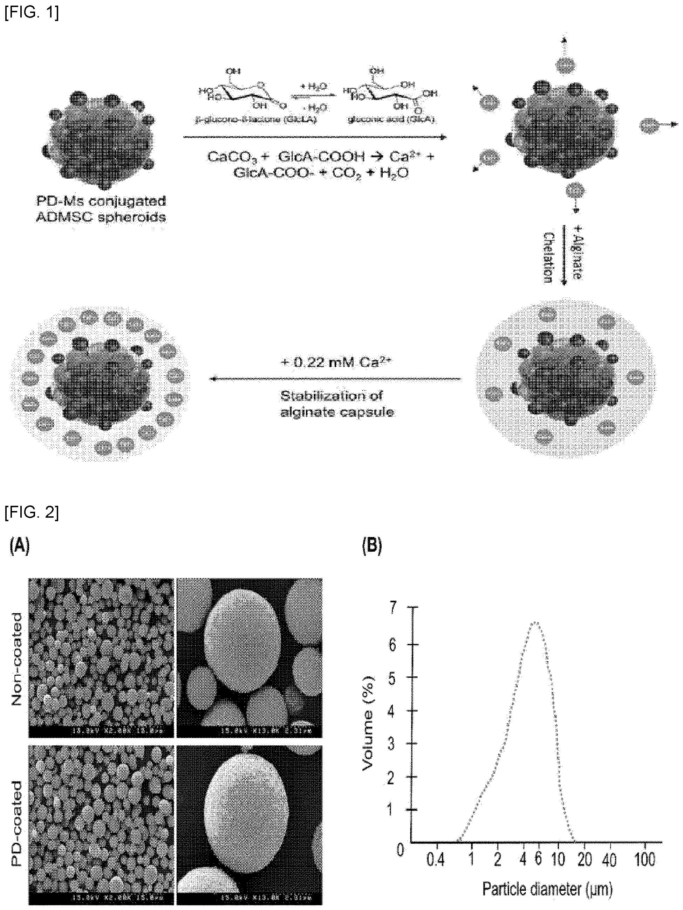

FIG. 1 shows a schematic diagram showing the process of forming a growth-type alginate gel for individual encapsulation of cells.
FIG. 2 shows a result of confirming the characteristics of calcium carbonate microspheres and polydopamine-coated calcium carbonate microspheres; FIG. 2A is a scanning electron microscope (SEM) image of calcium carbonate microspheres and polydopamine-coated microspheres; and FIG. 2B shows a result of confirming the size distribution of microspheres using a laser diffraction method.

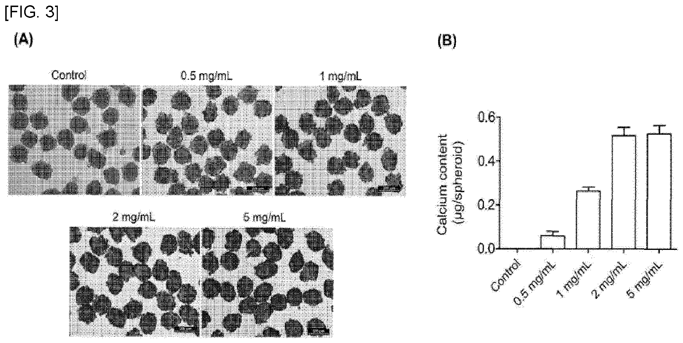

FIG. 3 shows optimization for the conjugation of adipose-derived mesenchymal stem cell spheroids (ADMSC spheroids) with PD-MS; FIG. 3A shows optical images of control ADMSC spheroids and ADMSC spheroids incubated with PD-MS suspension at concentrations of 0.5, 1, 2, and 5 mg/mL for 10 min; and FIG. 3B shows quantification of calcium content on the surface of ADMSCs after 10 min incubation with PD-MS suspension at concentrations of 0.5, 1, 2, 5 mg/mL.

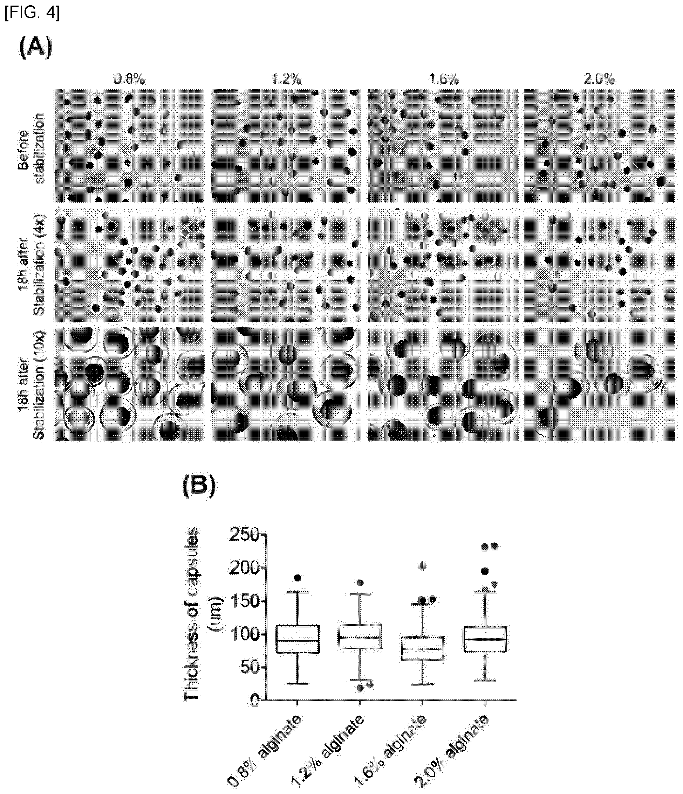

FIG. 4 shows a result of confirming the impact of alginate concentration on the formation of alginate shells; FIG. 4A shows optical images of ADMSC spheroids encapsulated using alginate solution at concentration of 0.8%, 1.2%, 1.6%, and 2.0%; and FIG. 4B shows thickness of alginate shells formed on the surface of ADMSC spheroids using alginate solution at a concentration of 0.8%, 1.2%, 1.6%, and 2.0%.

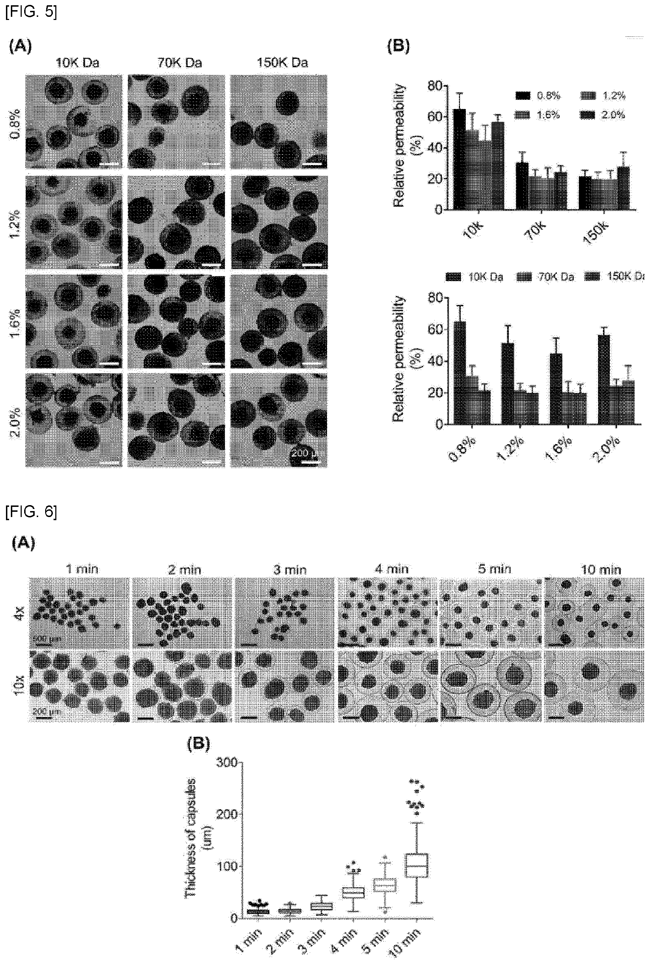

FIG. 5 shows a result of confirming the impact of gelation time on the formation of alginate shells; FIG. 5A shows optical images of encapsulated ADMSC spheroids after incubating PD-MS-ADMSC spheroids in 1.2% alginate solution for 1, 2, 3, 4, 5, and 10 min; and FIG. 5B shows thickness of alginate shells formed on the surface of ADMSC spheroids after 1, 2, 3, 4, 5, and 10 min of gelation.
FIG. 6 shows a result of confirming the selective permeability of alginate shells; FIG. 6A shows confocal images of encapsulated ADMSC spheroids immersed in dextran-FITC (MW: 10k, 70k, and 150k Da) for 3 h; and FIG.6B shows relative permeability of dextran-FITC with the molecular weight of 10k, 70k, and 150k Da into the alginate shells prepared using alginate at a concentration of 0.8%, 1.2%, 1.6%, and 2.0%.

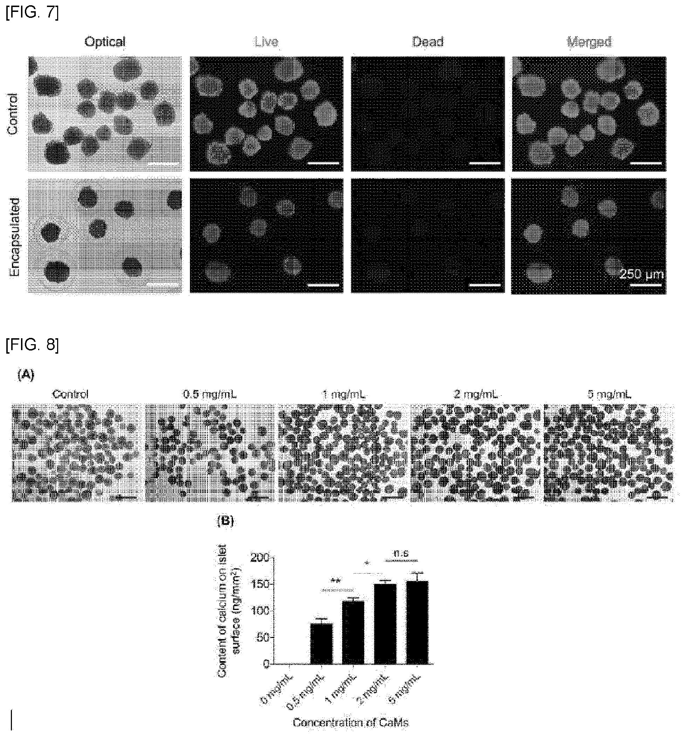

FIG. 7 shows a result confirming the viability of ADMSC spheroids before and after encapsulation assessed by LIVE/DEAD assay.
FIG. 8 shows a result of confirming the optimization for the conjugation of pancreatic islets with PD-MS; FIG. 8A shows optical images of control islets and islets incubated with PD-MS suspension at concentrations of 0.5, 1, 2, and 5 mg/mL for 10 min; and FIG. 8B shows quantification of calcium content on the surface of islets after 10 min of incubation with PD-MS suspension at concentrations of 0.5, 1, 2, 5 mg/mL.

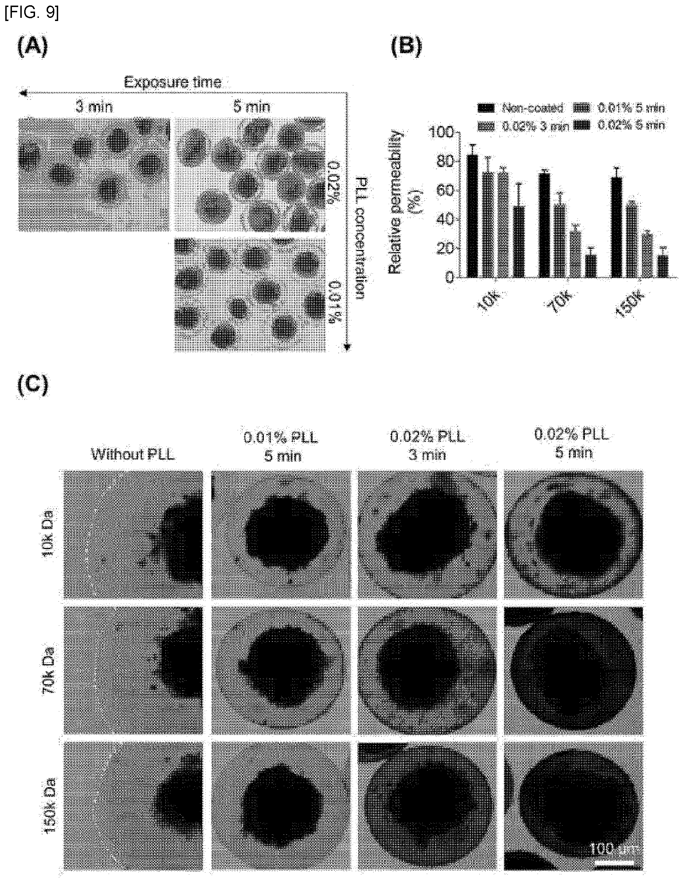

FIG. 9 shows a result of confirming the characteristics of alginate capsules after coating of poly-L-lysine; FIG. 9A shows optical images of alginate capsules after PLL coating; FIG. 9B shows relative permeability of dextran-FITC with different molecular weight (10k Da, 70k Da, and 150k Da) into alginate capsules; and FIG. 9C shows permeability of dextran-FITC into alginate capsules as assessed by CLSM

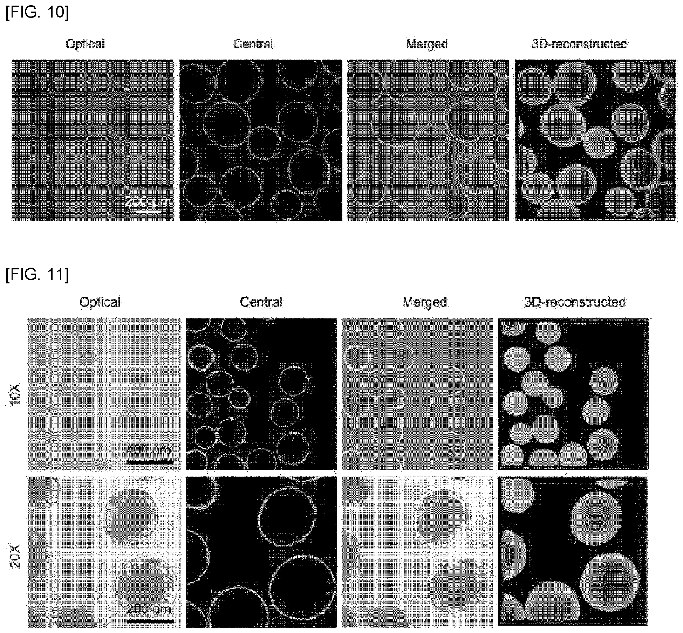

FIG. 10 shows a result showing the assessment of alginate coating on poly-L-lysine-coated alginate capsules using confocal laser scanning microscopy.
FIG. 11 shows a result of a confocal laser scanning microscope image of alginate coating in poly-L-lysine-coated alginate capsules.

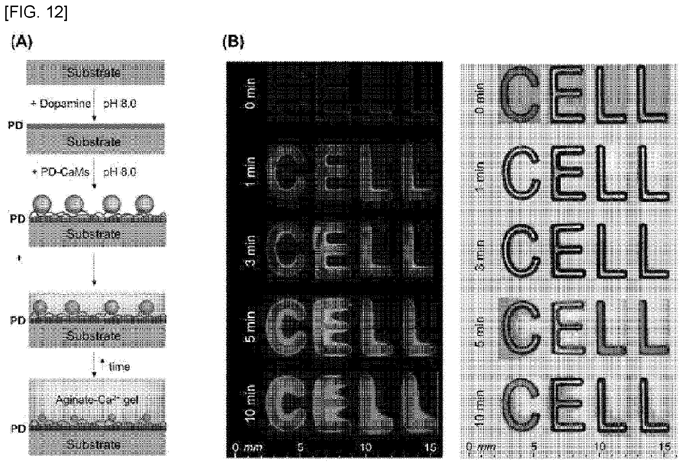

FIG. 12 shows a result of confirming versatility of surface-triggering in situ gelation (STIG) technology using alginate hydrogel; FIG. 12A shows methodology and growing mechanism for in situ gelation of alginate on various substrates.; and FIG. 12B shows time-dependent growth of alginate gel on the surface of 3D letters.

### [Best Mode]

Hereinafter, the present invention will be described in more detail.

The present inventors has confirmed that calcium carbonate microspheres conjugated to the surface of a spheroid containing a drug or a bioactive material release calcium ions in the alginate solution and chelate with alginate in the solution, thereby Individually microencapsulating drugs or bioactive materials by gradually forming alginate gel on the spheroid surface and thus a drug or a bioactive material is placed in the center of a capsule in a very simple way, and a capsule of a very small size can be manufactured in a short time compared to the conventional encapsulation method by adjusting the size of the capsule, and has completed the present invention.

Accordingly, the present invention provides a composition for microcapsules, which comprises: an object; microspheres conjugated to the object and composed of a material containing divalent cation; and alginate gel surrounding outside of the object and the microsphere, wherein the alginate gel is formed through a chelate bond between the divalent cation released from the material containing the divalent cation and alginate.

Preferably, the present invention provides a composition for microcapsules, which comprises: an object; calcium carbonate microspheres conjugated to the object; and alginate gel surrounding outside of the object and the microsphere, wherein the alginate gel is formed through a chelate bond between calcium ions released from the calcium carbonate and alginate.

The divalent cation may be selected from the group consisting of Pb²⁺, Cu²⁺, Cd2+, Ba²⁺, Sr²⁺, Ca²⁺, Co²⁺, Ni²⁺, Zn²⁺ and Mn²⁺, but it is not limited thereto.

The microspheres may be coated with polydopamine, but it is not limited thereto.

The object may be selected from the group consisting of cells, drugs, bioactive materials, polymers, metals and metal oxides, but it is not limited thereto.

The cells may be selected from the group consisting of pancreatic islet cells, mesenchymal stem cells, stem cells, chondrocytes, fibroblasts, osteoclasts, hepatocytes, cardiomyocytes, microbial cells, organoids, and cell spheroids, but it is not limited thereto.

The drug may be selected from the group consisting of immunosuppressants, anticoagulants, anti-inflammatory agents, antioxidants and hormones, but it is not limited thereto.

Preferably, the immunosuppressive agent may be one or more selected from the group consisting of Tacrolimus, Cyclosporin, Sirolimus, Everolimus, Ridaforolimus, Temsirolimus, Umirolimus Zotarolimus, Leflunomide, Methotrexate, Rituximab, Ruplizumab, Daclizumab, Abatacept and Belatacept, but it is not limited thereto.

Preferably, the anticoagulant may be at least one selected from the group consisting of argatroban, coumarin, heparin, low molecular weight heparin, hirudin, dabigatran, melagatran, clopidogrel, ticlopidine and absimab, but it is not limited thereto.

Preferably, the anti-inflammatory agent may be one or more selected from the group consisting of acetoaminephen, aspirin, ibuprofen, dicrofenac, indomethacin, piroxicam, fenoprofen, flubiprofen, ketoprofen, naproxen, suprofen, loxopropen, cinoxicam and tenoxicam, but it is not limited thereto.

The bioactive material may be selected from the group consisting of proteins, peptides, antibodies, genes, siRNAs, microRNAs and cells, but it is not limited thereto.

More specifically, the object may be selected from polystyrene, polyethylene, polypropylene, polycarbonate, polyethylene terephthalate, polyester, polydimethylsiloxane, polytetrafluoroethylene, polyethersulfone, polyvinyl alcohol, polyvinyl alcohol/poly acrylic acid, polyvinylidenefluoride, polyetheretherketone, polyurethane, polylactic-co-glycolic acid, polycaprolactone, polyimide, polydopamine capsules and nylon as polymers; cellulose, paper and silk as natural polymers; graphene, graphene oxide, graphene nanotubes, diamond and diamond-like carbon as carbon materials; clay, quartz, fertilizer, mica, hydroxyapatite, calcium phosphate and calcium carbonate as minerals; Si₃N₄ and tetraethyl orthosilicate as silicates; SiO₂, glass and CdS/CdSe as glass; GaAs and In₂O₃/SnO₂ as new materials; Pd, Pt, Cu, Ag, Fe and Al as metals; TiO₂, ZrO₂, Nb₂O₃, Fe₃O₄, ZnO₂ and Al₂O₃ as metal oxides; Al(OH)₃ as metal hydroxide; stainless steel as an alloy; viruses, *E. coli*, a superhydrophobic surface and a water surface as a living surface, but it is not limited thereto.

The average diameter of the microcapsules may be 0.05 to 20 µm, but it is not limited thereto.

In addition, the present invention provides a method of preparing microcapsules comprising: preparing microspheres composed of a material containing divalent cation (first step); coating polydopamine on the surface of the calcium carbonate microspheres by mixing a solution in which the calcium carbonate microspheres are suspended and a dopamine solution (second step); conjugating the polydopamine-coated calcium carbonate microspheres (PD-MS) to the surface of the object (third step); and coating the surface of the object to which the PD-MS is conjugated with an alginate gel (fourth step).

Preferably, the present invention provides a method of preparing microcapsules comprising: preparing calcium carbonate microspheres by mixing a calcium chloride solution and a sodium carbonate solution and then drying (first step); coating polydopamine on the surface of the calcium carbonate microspheres by mixing a solution in which the calcium carbonate microspheres are suspended and a dopamine solution (second step); conjugating the polydopamine-coated calcium carbonate microspheres (PD-MS) to the surface of the object (third step); and coating the surface of the object to which the PD-MS is conjugated with an alginate gel (fourth step).

The divalent cation may be selected from the group consisting of Pb²⁺, Cu²⁺, Cd²⁺, Ba²⁺, Sr²⁺, Ca²⁺, Co²⁺, Ni²⁺, Zn²⁺ and Mn²⁺, but it is not limited thereto.

In the second step, the microsphere surface may be coated with polydopamine by mixing 40 to 60 parts by weight of the microsphere suspension and 40 to 60 parts by weight of the dopamine solution.

In the third step, polydopamine-coated microspheres (PD-MS) was mixed with the object at a concentration of 1 to 4 mg/mL.

In the fourth step, the PD-MS-conjugated spheroids was immersed in 1-1.5 wt% of alginate solution and incubated for 5-15 minutes.

The alginate solution may further comprise D-(+)-gluconic acid-δ-lactone.

The object may be selected from the group consisting of cells, drugs, bioactive materials, polymers, metals and metal oxides, but it is not limited thereto.

In addition, the present invention provides an individual encapsulation method of an object comprising: preparing microspheres composed of a material containing divalent cation (first step); coating surface of the microspheres with polydopamine by mixing a microspheres solution in which the microspheres are suspended and a dopamine solution (second step); conjugating polydopamine-coated microspheres (PD-MS) to surface of an object (third step); and coating surface of PD-MS-conjugated object with an alginate gel (fourth step).

Preferably, the present invention provides an individual encapsulation method of an object comprising: preparing calcium carbonate microspheres by mixing a calcium chloride solution and a sodium carbonate solution and then drying (first step); coating polydopamine on the surface of the calcium carbonate microspheres by mixing a solution in which the calcium carbonate microspheres are suspended and a dopamine solution (second step); conjugating the polydopamine-coated calcium carbonate microspheres (PD-MS) to the surface of the object (third step); and coating the surface of the object to which the PD-MS is conjugated with an alginate gel (fourth step).

The divalent cation may be selected from the group consisting of Pb²⁺, Cu²⁺, Cd²⁺, Ba²⁺, Sr²⁺, Ca²⁺, Co²⁺, Ni²⁺, Zn²⁺ and Mn²⁺, but it is not limited thereto.

Hereinafter, the present invention will be described in more detail through examples. These examples are only intended to illustrate the present invention in more detail, and it will be apparent to those skilled in the art that the scope of the present invention is not limited by these examples according to the gist of the present invention. The examples of the present invention are provided to more completely explain the present invention to those of ordinary skill in the art.

The following experimental examples are intended to provide experimental examples commonly applied to each example according to the present invention.

### <Experimental Example 1> Checking calcium content of cell surface

To check the calcium content in the cell-particle complex, 30 ADMSC spheroids or pancreatic islets were washed three times with calcium-free buffer and immersed in 200 µL of saline containing 3.7% hydrochloric acid for 10 minutes. Then, using a calcium colorimetric assay kit (Biovision, Milpitas, MA) according to the manufacturer's protocol, the calcium content in the supernatant was quantified.

### <Experimental Example 2> Confirmation of characteristics of alginate capsules

Encapsulated ADMSC spheroids or pancreatic islets were identified using an optical microscope (Eclipse Ti, Nikon, Tokyo, Japan). The thickness of the alginate capsule was confirmed with about 200 spheroids or pancreatic islets using NIS Element BR software (Nikon, Tokyo, Japan), and Turkey load box and whisker plot using GraphPad Prism 5 software (GraphPad Software, CA) data is shown.

### <Example 1> Preparation and confirmation of polydopamine-coated calcium carbonate microspheres (PD-MS)

Calcium carbonate microspheres (MS) were successfully prepared by a simple mixing method in which a calcium chloride solution and a sodium carbonate solution were vigorously stirred.

### 1. Preparation of calcium carbonate microspheres

Calcium carbonate particles were fabricated by the ionic exchange reaction between calcium chloride and sodium carbonate.

Briefly, 0.5 mL of calcium chloride solution (0.33 M) and 0.5 mL of sodium carbonate solution (0.33 M) were mixed in 1 mL E-tubes and vigorously vortexed for 1 min.

The particles were collected by centrifugation at 1000 rpm and washed 3 times with distilled water and 2 times with acetone. Finally, the samples were kept at room temperature overnight for drying.

### 2. Surface modification of calcium carbonate with polydopamine

Calcium carbonate microspheres were coated with a thin layer of polydopamine membrane by self-polymerization in weak alkaline condition.

In brief, calcium carbonate microsphere suspension (1 mg/mL) in bicarbonate buffer (pH = 8.5; 10 mM) was mixed with a same volume of dopamine in bicarbonate buffer (pH 8.5; 10 mM).

The mixture was then allowed to stir uncovered at room temperature for 1 h.

PD-MS (polydopamine-functionalized calcium carbonate microspheres) was purified from free dopamine and free particulates of PD by applying 3 cycles of centrifugation at 2000 rpm for 5 min and reconstitution with distilled water.

The gathered PD-MS were lyophilized and stored at -20 °C for further experiments.

### 3. Confirmation of prepared polydopamine-coated calcium carbonate microspheres

To confirm the polydopamine-coated calcium carbonate microspheres prepared by the above process, microspheres (MS) and PD-MS were fixed on a brass tube using 2-side adhesive tape, and the samples were coated with a thin layer of platinum using an Ion Sputter system (E-1030; Hitachi, Tokyo, Japan) and observed under a scanning electron microscope (SEM; S-4100; Hitachi, Tokyo, Japan).

The SEM images depicted discrete microspheres with the size of 2-10 µm (FIG. 2A), which was consistent with the dynamic size measure by laser diffraction (FIG. 2B).

In addition, during the incubation of MS with dopamine in HBSS pH 8.5, the mixture manifested a progressive color change over time from white to black. In addition, SEM images showed the significant increase of the roughness of MS surface after 1 h of incubation, suggesting the success coating of polydopamine on the surface of MS (FIG. 2A).

From the above results, it was confirmed that the coating of polydopamine on the MS surface was successfully performed.

### <Example 2> Preparation and confirmation of PD-MS conjugated ADMSC spheroids

### 1. Preparation of spheroids from adipose-derived mesenchymal stem cells (ADMSCs)

Hanging drop method was used for fabrication of adipose-derived mesenchymal stem cell (ADMSC) spheroids. Briefly, ADMSCs were detached by trypsinization and suspended in α-MEM supplemented with 10% (v/v) of FBS and 1% (v/v) of antibiotics-antimycotics. Then, 25 µL of suspension containing 1000 ADMSCs was used to make a drop on the inside of a lid of a culture dish.

Sterile water was added into the dish to reduce the evaporation of the liquid in the drops. After 3 days of incubation in a humidified atmospheres containing 5% CO₂ at 37 °C, ADMSC spheroids were collected using a sterile capillary tube. Size and morphology of spheroids were assessed using a light microscope (Eclipse Ti, Nikon, Tokyo, Japan).

### 2. Immobilization of PD-MS on ADMSC spheroid surface

The conjugation of PD-MS with ADMSC spheroids was performed under a weak alkaline condition.

Prior to the conjugation, approximately 200 ADMSC spheroids were washed twice with Hank's balanced salt solution (HBSS; pH 8.0; without Mg²⁺and Ca²⁺) and pelleted in 1.5 mL microtubes (Axygen; Corning, NY).

After that, 1 mL of PD-MS suspension (2 mg/mL) and 100 cell clusters were added to each tube, left to incubate at 37 °C for 10 min with gentle inversion every 1 min to immobilize PD-MS on the surface of the ADMSC spheroids.

The ADMSC spheroids were then collected and transferred to a culture disk containing 10 mL of culture medium. The ADMSC spheroids were further purified from unbound PD-MS by handpicking using a micropipette.

### 3. Characterization of PD-MS conjugated ADMSC spheroids

In order to confirm the characteristics of the ADMSC spheroids prepared by the gravity-mediated aggregation method as described above, the mean diameter of the obtained cell cluster was confirmed.

As showed in FIG. 3A, the deposition of black PD-MS particles was clearly observed on the surface of cell clusters after incubation, and notably, the density of particles was increased in accordance with the increase of particle concentration.

In addition, the content of calcium on the surface of cell clusters incubated with PD-MS at concentration of 0.5 mg/mL, 1 mg/mL, 2 mg/mL, 5 mg/mL were determined to be 0.0590 ± 0.0373 µg/spheroid, 0.2550 ± 0.0410 µg/spheroid, 0.5478 ± 0.0507 µg/spheroid, 0.5261 ± 0.0651 µg/spheroid, respectively.

The data clearly indicated that the immobilization of PD-MS on the surface of ADMSC spheroids reached saturation when the PD-MS were used at a concentration of 2 mg/mL.

On the other hand, higher particle concentration (5 mg/mL) resulted in unstable binding of particle and formation of large particle clusters, making purification step more difficult. Thus, we selected PD-MS concentration at 2 mg/mL for further experiments.

### <Example 3> Optimization for encapsulation of PD-MS-conjugated ADMSC spheroids

### 1. Encapsulation process

For the formation of alginate shells, PD-MS conjugated ADMSC spheroids were suspended in alginate (Keltone HVCR, FMC Polymer) solution containing D-(+)-gluconic acid-δ-lactone as the acidifier.

The sustained hydrolysis of D-(+)-gluconic acid-δ-lactone gradually reduces pH of the solution, triggering the release of calcium for the formation of alginate gel on the surface of spheroids.

After that, ADMSC spheroids were picked up using a 1-mL pipette and washed 3 times with calcium-free saline. Finally, ADMSC spheroids were transferred into saline containing calcium (22 mM) to stabilize the alginate capsules.

The impacts of different variables of encapsulation process using different alginate concentration (0.8%, 1%, 1.2%, 2%), gelation time (1, 2, 3, 4, 5, and 10 min), concentration of D-(+)-gluconic acid-δ-lactone, concentration of calcium on the thickness and permeability of the alginate shells were investigated to optimize the encapsulating process.

### 2. Effect of alginate concentration on formation of alginate capsules

To determine the impact of alginate concentration on the formation of alginate capsules, different concentrations (0.8%, 1.2%, 1.6%, and 2.0%) of alginate were used for the encapsulation of ADMSC spheroids.

As a result, as shown in FIG. 4A, at alginate concentration of 1.2%, all of ADMSC spheroids were individually encapsulated in faultless spherical alginate capsules, respectively. On the other hand, at a lower concentration (0.8%), a notable percentage of capsules possessed hemispherical morphology.

The possible reason could be that at lower alginate concentration, ADMSC spheroids quickly settled down on the bottom of the tubes due to lower viscosity of alginate solution. Thus, spheroids were not completely exposed to the alginate solution for the gelation, resulting in the asymmetric formation of alginate capsules.

On the other hand, the increase of alginate concentration (1.6%, 2%) resulted in the increased percentages of capsules containing more than one spheroids.

It could be explained that the increase of solution viscosity might interfere with the physical force during suspending ADMSC spheroids in alginate capsules. Thus, it induced a dissimilar distribution of ADMSC spheroids, resulting in a higher chance for formation of capsules containing more than one spheroids.

In addition, the thicknesses of capsules prepared using alginate concentration at 0.8%, 1.2%, 1.6%, and 2% were 61.38 ± 29.73 µm, 63.93 ± 15.95 µm, 52.95 ± 16.74 µm, and 62.65 ± 19.75 µm, respectively (FIG. 4B). There was a non-significant difference between these values in all group, suggesting the negligible impact of alginate concentration on the thicknesses of alginate capsules.

From the above results, it could be confirmed that the shape of the alginate capsule is very important for high encapsulation efficiency, and accordingly, it was confirmed that the alginate concentration at 1.2% has the highest encapsulation efficiency and better morphology of alginate capsules.

### 3. Effect of incubation time on formation of alginate capsules

To determine the impact of the incubation time on the formation of alginate capsules. ADMSC spheroids conjugated with PD-MS were incubated in 1.2% alginate solution for different periods (1 min, 2 min, 3 min, 4 min, 5 min, 10 min).

As a result, FIG. 5A unambiguously demonstrated the increase of capsule thickness in a time-dependent manner. The thicknesses of the capsules formed after 1 min, 2 min, 3 min, 4 min, 5 min, and 10 min of incubation were 13.15 ± 4.50 µm, 14.99 ± 4.67 µm, 23.68 ± 7.67 µm, 49.55 ± 13.52 µm, 63.93 ± 15.95 µm, and 104.86 ± 36.32 µm, respectively.

As mentioned above, the sustained hydrolysis of D-(+)-gluconic acid-δ-lactone gradually reduces pH of the solution, triggering the release of calcium for the formation of alginate gel on the surface of spheroids. Thus, increase incubation time boosted the release and diffusion of calcium ions into the surrounding alginate solution, resulting in the formation of a thicker layer of alginate gel.

From the above results, it was confirmed that the encapsulation method of the present invention provides tunable thicknesses of encapsulating capsules by simply adjusting the incubation time of PD-MS conjugated cell spheroids in alginate solution.

### <Example 4> Selective permeability of alginate capsules

The main purpose of cell microencapsulation is to provide a semipermeable membrane which allows the free ingress of oxygen, nutrients, and therapeutic molecules while reducing the diffusion of antibodies. Thus, strict control over the permeability of the alginate shells is required for effective immunoprotective effect and maintenance of cell function.

Accordingly, FITC-labeled dextran was used as molecular weight standards to assess the permeability of alginate shells. The use of neutral dextran was reported to prelude the issues related to the absorption, aggregation, and other charge/hydrophobic interactions (Briššová, Petř̌̌o, Lacík, Powers, & Wang, 1996). The fluorescent intensities inside the capsules and in the surrounding solution were measured for individual capsule using a confocal laser scanning microscope.

In vitro permeability assay was conducted to evaluate the ingress ratio of macromolecular markers using FITC-dextran (MW: 10k, 70k, and 150k Da) as fluorescent molecular weight standards. Approximately 50 encapsulated islets were immersed in 1 mL of 0.1% FITC-dextran solution in PBS for 3 h.

Then, the 100 µL of a solution containing encapsulated islets were placed in a glass-bottom confocal dish (SPL Life Sciences, Gyeonggi, Korea) and observed under a confocal laser scanning microscope (CLSM, Leica Microsystems, Wetzlar, Germany).

Mean pixel grey values representing the relative fluorescent intensities inside the capsules and in the surrounding buffer were measured using ImageJ software. The diffusion of FITC-dextran into capsules was expressed as the percentage of fluorescent intensity in the microcapsules confines relative to that in the surrounding solution.

FIG. 6A depicted the signification reduction of dextran ingress when the molecular weight increased. Low molecular weight dextran easily diffused to the interior of the capsules with ingress ration more than 50%. Meanwhile, the permeation of higher molecular weight dextran (70k Da and 150k Da) was greatly attenuated, reflected by the ingress ratio of approximately 20% (FIG. 6B and FIG. 6C).

### <Example 5> Viability of encapsulated ADMSC spheroids

To determine the effect of alginate encapsulation process on cell viability, membrane integrity staining analysis of unencapsulated and encapsulated spheroids was performed using acridine orange (AO; Sigma, St. Louis, MO) and propidium iodine (PI; Sigma, St. Louis, MO) before and after encapsulation. The viability of ADMSC spheroids was confirmed.

AO and PI were dissolved in α-MEM at a concentration of 0.67 µM and 75 µM, respectively and incubated with cell spheroids for 5 min under light protection. Green and red fluorescence in cell spheroids were then recorded using a fluorescent microscope (Eclipse Ti, Nikon, Tokyo, Japan).

Since AO is cell permeable, all stained nucleated cells generate green fluorescence. PI only enters the cells with compromised membranes, and therefore, dying, dead, and necrotic cells only fluoresce red (Bank, 1988).

As a result, as shown in FIG. 7, no significant difference between red and green fluorescent intensities in ADMSC spheroids before and after encapsulation.

From the above results, it was confirmed that the cell viability was maintained during the formation of alginate capsules.

### <Example 6> Preparation and encapsulation of pancreatic islets conjugated with PD-MS, and confirmation of properties thereof

### 1. Immobilization of PD-MS on surface of pancreatic islets

The conjugation of PD-MS with pancreatic islets was performed under a weak alkaline condition.

Prior to the conjugation, approximately 100 pancreatic islets were washed twice with Hank's balanced salt solution (HBSS; pH 8.0; without Mg²⁺ and Ca²⁺) and pelleted in 1.5 mL microtubes (Axygen; Corning, NY).

After that, 1 mL of PD-MS suspension (2 mg/mL) was added to each tube, left to incubate at 37 °C for 10 min with gentle inversion every 1 min to immobilize PD-MS on the surface of the pancreatic islets.

The pancreatic islets were then collected and transferred to a culture disk containing 10 mL of culture medium. The pancreatic islets were further purified from unbound PD-MS by handpicking using a micropipette.

### 2. Characterization of PD-MS conjugated pancreatic islets

PD-MS were immobilized on the surface of islets by simply mixing 1 mL of PD-MS suspension with 100 islets for 10 min.

As showed in FIG. 8A, the deposition of black PD-MS particles was clearly observed on the surface of islets after incubation, and notably, the density of particles was increased in accordance with the increase of particle concentration

In addition, the content of calcium on the surface of islets incubated with PD-MS at concentration of 0.5 mg/mL, 1 mg/mL, 2 mg/mL, 5 mg/mL were determined to be 75.4566 ± 22.6963 ng/mm² surface, 117.5974 ± 15.2445 ng/mm² surface, 149.0031 ± 18.0960 ng/mm² surface, 154.9235 ± 36.6842 ng/mm² surface, respectively.

The data clearly indicated that the immobilization of PD-MS on the surface of islets reached saturation when the PD-MS were used at a concentration of 2 mg/mL.

On the other hand, higher particle concentration (5 mg/mL) resulted in unstable binding of particle and the formation of large particle clusters, making purification step more difficult. Thus, PD-MS concentration at 2 mg/mL was selected for further experiments.

### 3. Synthesis of fluorescence-labeled alginate (F-alginate)

Briefly, 500 mg of alginate was dissolved in 100 mL of distilled water. Then, 24.64 mg EDC (1-Ethyl-3-(3-dimethylaminopropyl)carbodiimide; Tokyo Chemical Industry Co., Ltd, Tokyo, Japan), 29.60 mg NHS (N-hydroxysccinimide; Tokyo Chemical Industry Co., Ltd, Tokyo, Japan), 89.284 mg fluoresceinamine (Tokyo Chemical Industry Co., Ltd, Tokyo, Japan) were added to the solution and stirred at room temperature for 6 h. F-alginate was precipitated by mixing 1 volume of reactants with 9 volume of ice cold absolute alcohol. The pellets were washed with absolute alcohol until the supernatant became colorless. The samples were lyophilized and stored at -20°C until use.

### 4. Encapsulation of PD-MS conjugated pancreatic islets

For the formation of alginate shells, PD-MS conjugated islets were suspended in alginate (Keltone HVCR, FMC Polymer) solution containing D-(+)-gluconic acid-δ-lactone (20 mg/ml) as the acidifier.

The sustained hydrolysis of D-(+)-gluconic acid-δ-lactone gradually reduces pH of the solution, triggering the release of calcium for the formation of alginate gel on the surface of islets.

After that, islets were picked up using a 1-mL pipette and washed 3 times with calcium-free saline. Finally, islets were transferred into saline containing calcium (22 mM) to stabilize the alginate capsules.

The impacts of different variables of encapsulation process such as alginate concentration (0.8%, 1%, 1.2%, 2%), gelation time (1, 2, 3, 4, 5, and 10 min), concentration of D-(+)-gluconic acid-δ-lactone, concentration of calcium on the thickness and permeability of the alginate shells were investigated to optimize the encapsulating process.

### 5. Coating of poly-L-lysine and alginate on surface of capsules

Alginates, known as negatively charged polymers, are able to form strong complexes with polycations such as poly(L-lysine), poly (L-ornithine), poly(ethylene imine). As these complexes are stable in physiological condition, they have been extensively used to stabilize and control the porosity of alginate capsules. Accordingly, in the present invention, a layer of PLL was coated on the surface of alginate shells by incubating alginate-coated islets in poly-L-lysine solution.

Alginate capsules were washed 3 times with saline and incubated in 100 mM CaCl₂ solution. After that, the capsules were washed twice with mannitol (0.3 M). The PLL (MW: 12k Da; Sigma-Aldrich, MO) solution in saline at various concentrations (0.01%, 0.02%) was added to capsules; the mixture was then incubated for 5 min under slight agitation at 37°C. Free PLL was removed by washing the capsules twice with saline and twice with full media. The morphology of alginate capsules were observed under a light microscope (Eclipse Ti, Nikon, Tokyo, Japan). To observe the coating of coverage of PLL, PLL was labeled with FITC and the PLL-coated capsules were assessed under a confocal laser scanning microscope (CLSM, Leica Microsystems, Wetzlar, Germany).

A second layer of alginate was coated on the surface of PLL-coated alginate capsules by electrostatic interaction. PLL-coated capsules were incubated in an alginate solution (0.02%) in saline for 5 min under slight agitation for every 30s. Finally, the capsules were washed twice with saline and twice with full media.

As a result, as shown in FIG. 9A, it was confirmed that the optimal PLL concentration and incubation time were 0.02% and 3 min, respectively.

### 6. Confirmation of selective permeability of alginate capsules

In vitro permeability assay were conducted to evaluate the ingress ratio of macromolecular markers using FITC-dextran (MW: 10k, 70k, and 150k Da) as fluorescent molecular weight standards. Approximately 50 encapsulated pancreatic islets were immersed in 1 mL of 0.1% FITC-dextran solution in PBS for 3 h.

Then, the 100 µL of a solution containing encapsulated pancreatic islets were placed in a glass-bottom confocal dish (SPL Life Sciences, Gyeonggi, Korea) and observed under a confocal laser scanning microscope (CLSM, Leica Microsystems, Wetzlar, Germany).

Mean pixel grey values representing the relative fluorescent intensities inside the capsules and in the surrounding buffer were measured using ImageJ software. The diffusion of FITC-dextran into capsules was expressed as the percentage of fluorescent intensity in the microcapsules confines relative to that in the surrounding solution.

As a result, as the molecular weight increased, as shown in FIG. 9B and FIG. 9C, a significant decrease in dextran penetration was confirmed, and it was confirmed that the penetration rate of high molecular weight dextran (70k Da and 150k Da) was significantly reduced.

### 7. Viability of encapsulated pancreatic islets

To determine the effect of alginate encapsulation process on cell viability, the viability of pancreatic islets was confirmed before and after encapsulation with acridine orange (AO; Sigma, St. Louis, MO) and propidium iodine (PI; Sigma, St. Louis, MO).

AO and PI were dissolved in α-MEM at a concentration of 0.67 µM and 75 µM, respectively and incubated with cell spheroids for 5 min under light protection. Green and red fluorescence in pancreatic islets were then recorded using a fluorescent microscope (Eclipse Ti, Nikon, Tokyo, Japan).

As a result, as shown in FIG. 10, it was confirmed that the PLL was limited only to the outer surface of the alginate shell, thereby minimizing the toxicity caused by direct contact between the PLL and the cell.

However, it has been reported that PLL exhibits immunogenicity by increasing host cell binding and promoting the secretion of various cytokines that can impair cell survival and function. Therefore, a second layer of alginate was introduced on the surface of the alginate shell coated with PLL to improve compatibility.

As a result, FIG. 11 showed the complete coverage of alginate on the exterior surface of alginate coated with PLL after encapsulated pancreatic islets were incubated in alginate solution (0.02%) for 5 min.

### 8. 3D Character temperament preparation

Apart from the ability to encapsulate living cells, the versatility of the surface-triggering in situ gelation (STIG) technology can be used for therapeutic purposes by facilitating the surface modification of various materials. For example, coating of a thin alginate gel layer on the surface of substrates can reduce host immune response or change the wettability of the substrate, thus, improving its biocompatibility. In addition, drug delivery system/cell-laden hydrogel could be introduced on substrate surface for therapeutic purpose.

Therefore, in the present invention, 3D letters, which includes "C", "E" and "L" with the thickness, width, height of 1 mm, 2 mm, and 4 mm, respectively, were prepared with a commercialized resin (Stratasys VeroClear (RGD810) using PolyJet technology.

### 9. Conformal coating of alginate on surface of 3D letter objects

The 3D letters were washed 3 times by immersing in bicarbonate buffer (pH 8.5; 10 mM) and sonicating for 10 min. The 3D letters were then incubated with a dopamine solution (1 mg/mL) in bicarbonate buffer (pH 8.5; 10 mM) under stirring at room temperature for 1 h. After that, the 3D letters were washed 3 times with bicarbonate buffer and incubated in collagen solution (0.03 mg/mL) in bicarbonate buffer (pH 8.5; 10 mM) for 1 h. The samples were washed 3 times with bicarbonate buffer to remove free collagen. Then, a PD-CaMs suspension (2 mg/mL) in HBSS pH 8.0 was gently mixed with 3D letters for 20 min at room temperature. The 3D letters were washed 3 times with saline to remove unbound PD-CaMs. The modified letters were then immersed in an F-alginate solution (1.2%) in saline containing D-(+)-gluconic acid-δ-lactone (20 mg/mL). The mixture was rotated at 1 rpm and the formation of alginate layer on the surface of the letters at predetermined time intervals (1 min, 3 min, 5 min, 10 min) was evaluated using a fluorescence microscope (Eclipse Ti, Nikon, Tokyo, Japan).

The results clearly demonstrated the continuous growth of alginate hydrogel on the surface of 3D letters on a time-dependent manner (FIG. 12). The STIG technology can be employed for conformal coating of 3D complex structure, where the bulk hydrogel polymerization method is difficult to apply.

While the present invention has been particularly described with reference to specific embodiments thereof, it is apparent that this specific description is only a preferred embodiment and that the scope of the present invention is not limited thereby to those skilled in the art. That is, the practical scope of the present invention is defined by the appended claims and their equivalents.

## Claims

1. A composition for microcapsules, which comprises:
an object;
microspheres conjugated to the object and composed of a material containing divalent cation; and
alginate gel surrounding outside of the object and the microsphere,
wherein the alginate gel is formed through a chelate bond between the divalent cation released from the material containing the divalent cation and alginate.

2. The composition for microcapsules of claim 1, wherein the divalent cation is selected from the group consisting of Pb²⁺, Cu²⁺, Cd²⁺, Ba²⁺, Sr²⁺, Ca²⁺, Co²⁺, Ni²⁺, Zn²⁺ and Mn²⁺.

3. The composition for microcapsules of claim 1, wherein the microspheres are coated with polydopamine.

4. The composition for microcapsules of claim 1, wherein the object is selected from the group consisting of cells, drugs, bioactive material, metals and metal oxides.

5. The composition for microcapsules of claim 4, wherein the cells are pancreatic islet cells, mesenchymal stem cells, stem cells, chondrocytes, fibroblasts, osteoclasts, hepatocytes, cardiomyocytes, microbial cells, organoids, and cell spheroids.

6. The composition for microcapsules of claim 4, wherein the drugs are selected from the group consisting of immunosuppressants, anticoagulants, anti-inflammatory agents, antioxidants and hormones.

7. The composition for microcapsules of claim 4, wherein the bioactive materials are selected from the group consisting of proteins, peptides, antibodies, genes, siRNAs, microRNAs and cells.

8. The composition for microcapsules of claim 1, wherein the microcapsules have an average diameter of 0.05 to 20 µm.

9. A method of preparing microcapsules comprising:
preparing microspheres composed of a material containing divalent cation (first step);
coating surface of the microspheres with polydopamine by mixing a microspheres solution in which the microspheres are suspended and a dopamine solution (second step);
conjugating polydopamine-coated microspheres (PD-MS) to surface of an object (third step); and
coating surface of PD-MS-conjugated object with an alginate gel (fourth step).

10. The method of preparing microcapsules of claim 9, wherein the divalent cation is selected from the group consisting of Pb²⁺, Cu²⁺, Cd²⁺, Ba²⁺, Sr²⁺, Ca²⁺, Co²⁺, Ni²⁺, Zn²⁺ and Mn²⁺.

11. The method of preparing microcapsules of claim 9, wherein in the second step, 40 to 60 parts by weight of the microsphere suspension and 40 to 60 parts by weight of the dopamine solution are mixed to coat the surface of the microspheres with the polydopamine.

12. The method of preparing microcapsules of claim 9, wherein in the third step, the polydopamine-coated microspheres (PD-MS) are mixed with the object at a concentration of 1 to 4 mg/mL.

13. The method of preparing microcapsules of claim 9, wherein in the fourth step, the PD-MS-conjugated object is immersed in 1 to 1.5 wt% of alginate solution and incubated for 5 to 15 minutes.

14. The method of preparing microcapsules of claim 13, wherein the alginate solution further comprises D-(+)-gluconic acid-δ-lactone.

15. The method of preparing microcapsules of claim 9, wherein the object is selected from the group consisting of cells, drugs, bioactive materials, metals and metal oxides.

16. An individual encapsulation method of an object comprising:
preparing microspheres composed of a material containing divalent cation (first step);
coating surface of the microspheres with polydopamine by mixing a microspheres solution in which the microspheres are suspended and a dopamine solution (second step);
conjugating polydopamine-coated microspheres (PD-MS) to surface of an object (third step); and
coating surface of PD-MS-conjugated object with an alginate gel (fourth step).

17. The individual encapsulation method of an object of claim 16, wherein the divalent cation is selected from the group consisting of Pb²⁺, Cu²⁺, Cd²⁺, Ba²⁺, Sr²⁺, Ca²⁺, Co²⁺, Ni²⁺, Zn²⁺ and Mn²⁺.
